# EUROPEAN PATENT APPLICATION

(11) **EP 4 180 087 A1**
(43) Date of publication of application: **17.05.2023**
(21) Application number: 21208260.6
(22) Date of filing: 15.11.2021
(51) Int. Cl.: A61N 5/06, G16H 20/40

(54) **METHOD FOR ADJUSTING AN ACTUAL WAKE-UP TIME OF AN INDIVIDUAL**

(71) Applicant: BrainLit AB, 223 63 Lund (SE)
(72) Inventor: Singvall, Jakob, 237 34 Bjärred (SE); Wingren, Tord, 222 36 Lund (SE); Lindoff, Bengt, 237 35 Bjärred (SE); Smolka, Jochen, 224 80 Lund (SE)
(74) Representative: AWA Sweden AB

(57) **Abstract**

The present invention relates to a method (300) for adjusting an actual wake-up time of an individual. The method (300) comprising: determining (S302) a target light profile for the individual, based on a target wake-up time and a circadian rhythm model of the individual, wherein the target light profile describes illuminance and spectrum of light to be received by the individual over time; controlling (S304) a light source, configured to illuminate the individual, based on the target light profile such that an accumulated amount of light received by the individual is aligned with the target light profile; determining (S308) the actual wake-up time at which the individual wakes up; and in response to the actual wake-up time being outside a predetermined time-span from the target wake-up time (S312): updating (S310) the target light profile based on a comparison of the actual wake-up time and the target wake-up time. The present invention further relates to a system (100) and a control unit (200) for adjusting an actual wake-up time of an individual.

## Description

### Technical field

The present invention relates to a method for adjusting an actual wake-up time of an individual. The present invention further relates to a control unit for adjusting an actual wake-up time of an individual and a system comprising the control unit.

### Background of the invention

Chronobiology, which is a field of biology that studies timing (e.g., periodic) processes and phenomena in living organisms, is becoming increasingly more important for understanding human physiology. For example, the circadian rhythm is an internal process of living organisms (e.g. humans) that regulates the sleep-wake cycle. The circadian rhythm typically repeats over a time period which, for most people, is slightly longer than 24 hours. It is also known that this cycle can be different between different individuals. For example, some persons are known to be "morning" persons, while other are "evening" persons. Hence, the behavior at a certain time of day for different individual typically varies. This is usually referred to as a person's chronotype.

An important part of chronobiology is the study of the impact of light on living organisms. As is commonly known within the field, the circadian rhythm of living organisms can be affected by light. Living organisms, for example humans and animals, typically have cells in their eyes which are sensitive to certain wavelengths of light. It has, for example, been found that exposing a person to bright light in the evening can postpone the time that person falls asleep. Recent studies have further found that light can affect a person's alertness and performance.

In light of the busy lives we live and how society is organized, there are many things that can affect our well-being in a bad way. For instance, our daily work often forces us to be at work between certain times, which puts a constraint on how our days can be adapted for our individual needs. Thus, there is a need for improved ways of optimizing a person's well-being.

### Summary of the invention

Our modern lifestyle typically forces us to be at work at a certain time. This time is likely not optimized towards the individual person's natural circadian rhythm. Hence, people tend to use an alarm to comply with their work schedule. The inventors of the present inventive concept have realized that a method and a device for controlling light exposure of an individual can be adapted to take into account wake-up times of the individual and adjust the circadian rhythm of the individual accordingly. Through that, the well-being, e.g. physical and mental health, of the individual can be improved. In other words, it facilitates for an enhancement of the well-being of the individual considering external constraints, e.g. the individual's schedule, which are typically not ideal for the individual.

According to a first aspect a method for adjusting an actual wake-up time of an individual is provided. The method comprising: determining a target light profile for the individual, based on a target wake-up time and a circadian rhythm model of the individual, wherein the target light profile describes illuminance and spectrum of light to be received by the individual over time; controlling a light source, configured to illuminate the individual, based on the target light profile such that an accumulated amount of light received by the individual is aligned with the target light profile; determining the actual wake-up time at which the individual wakes up; and in response to the actual wake-up time being outside a predetermined time-span around the target wake-up time: updating the target light profile based on a comparison of the actual wake-up time and the target wake-up time.

The wording "actual wake-up time" should, within the context of this disclosure, be construed as the time at which the individual wakes up. In contrast, the wording "target wake-up time" should be interpreted as the time the individual wants or needs to wake up. For example, the target wake-up time may be dependent on what time the individual needs to be at work or school.

The wording "target light profile" should, within the context of this disclosure, be construed as information relating to a preferred light dose or a preferred amount of light that the user should accumulate over time. The preferred light dose/preferred amount of light may be different for different wavelengths. The term "over time" may refer to a time period over which the individual should be exposed to a certain amount of light, such as a day, a week, a month etc. The target light profile may be periodical with a period of 24 hours. This may, e.g., be the case when the same target wake-up time is preferred each day. The target light profile may comprise information spanning more than 24 hours, e.g. when different target wake-up times are needed over a set of days. Examples are in the change from normal time to summer time/daylight savings time (or vice versa), or inter-continental flights when changing time-zone are needed. In such cases the target light profile may be adjusted over a set of days in order to gradually adapt the individual's circadian rhythm to the new time or time zone.

By the wording "based on" as in "controlling a light source, configured to illuminate the individual, based on the target light profile" it is hereby meant that properties of light emitted from the light source, such as illuminance and spectrum, are adjusted such that the individual over time receives an accumulated amount of light which is in line with the target light profile. The light source may be multiple light sources from which the individual receives light during a day, a week, a month, etc.

The pre-determined time-span may be a time-span around the target wake-up time in which the individual should wake-up. In other words, the predetermined time-span may be a time-span before, after, or both before and after the target wake-up time. The pre-determined time-span may allow for flexibility when it comes to how the actual wake-up time relates to the target wake-up time. The predetermined time-span may be a time period prior to and/or after the target wake-up time. The actual wake-up time being outside the predetermined time-span may be interpreted as the individual wakes up too early or too late in regard to the target wake-up time.

By the wording "based on" as in "updating the target light profile based on a comparison of the actual wake-up time and the target wake-up time" it is hereby meant that in a case where the actual wake-up time is after the target wake-up time, the target light profile is adjusted so that the target light profile may specify that a high circadian stimulation should be given to the individual earlier in the day, while in the opposite case, i.e. where the actual wake-up time is prior to the target wake-up time, the target light profile may be adjusted such that a high circadian stimulation should be given to the individual later in the day. By adjusting the circadian stimulation over the day, a time when the individual goes to sleep can be adjusted, and thereby also the actual wake-up time.

A possible associated advantage of the above described method is that the individual's natural circadian rhythm can be adjusted based on an external condition, i.e. the target wake-up time, which is likely not ideal for the individual's well-being, thereby enhancing the individual's well-being. It may further have the effect that forced wake-ups, e.g. the individual waking up to an alarm clock instead of by himself, can be avoided, due to the adjustment of the individual's circadian rhythm. The individual may thereby be prevented from waking up during a deep sleep phase, thereby further increasing the well-being of the individual.

In other words, the method allows for an adjustment of the individual's circadian rhythm by controlling the light that the individual is exposed to, such that it is adapted for a target wake-up time.

The method may further comprise, in response to the actual wake-up time being outside a further predetermined time-span and after the target wake-up time: adjusting the target wake-up time based on a comparison of the actual wake-up time and the target wake-up time, and wherein the updating of the target light profile may be based on a comparison of the actual wake-up time and the adjusted target wake-up time.

Put differently, if the individual wakes up too late in relation to the target wake-up time, and outside the further predetermined time-span (e.g. an allowable time-span), the target wake-up time may be adjusted for a following day. Further, the target light profile may be adjusted based on the comparison of the actual wake-up time and the adjusted target wake-up time. By adjusting the target wake-up time, an improved process of adjusting the actual wake-up time can be achieved. For instance, because the circadian rhythm can be gradually adjusted in increments, rather than in a large step, which may be hard to achieve and/or affect the well-being of the individual negatively. As the actual wake-up time gets closer to the target wake-up time, the target wake-up time may be adjusted back to the time at which it was originally.

The method may further comprise, in response to the actual wake-up time being outside the further predetermined time-span and prior to the target wake-up time: adjusting the target wake-up time based on a comparison of the actual wake-up time and the target wake-up time, and wherein the updating of the target light profile may be based on a comparison of the actual wake-up time and the adjusted target wake-up time. The same aspects and advantages as mentioned above may apply also to the act of adjusting the target wake-up time when the actual wake-up time is outside the further predetermined time-span and prior to the target wake-up time.

Adjusting the target wake-up time based on a comparison of the actual wake-up time and the target wake-up time may involve adjusting the target wake-up time so that it is closer to the actual wake-up time. The larger a difference between the target wake-up time and the actual wake-up time is, the closer the adjusted target wake-up time may be to the actual wake-up time. A smaller difference between the target wake-up time and the actual wake-up time may lead to a smaller adjustment of the target wake-up time. As a non-limiting example, the target wake-up time may be adjusted in intervals of 5, 10, 20, or 30 minutes.

The acts of controlling the light source, determining the actual wake-up time, and updating the target light profile may be repeated until the actual wake-up time is within the predetermined time-span from the target wake-up time. The act of adjusting the target wake-up time may also be repeated in the same manner. Put differently, the steps of the method may be performed multiple times over a number of days. For example, the act of controlling the light source may be performed during a day and determining the actual wake-up time may be performed in the morning after when the individual wakes up. Based on that, the act of updating the target light profile may be performed, in order to control the light source based on the updated target light profile during that day.

A possible associated advantage is that the adjustment of the actual wake-up time, and/or the individual's circadian rhythm, can be made in steps to not cause too great of a change for the individual.

The predetermined time-span may be a time-span prior to the target wake-up time. Put differently, the predetermined time-span may be a time interval leading up to the target wake-up time. In the case of a time-span prior to the target wake-up time, the later end point of the time-span may be the target wake-up time. In other words, the time-span may include the target wake-up time. Alternatively, the later end point of the time-span may be strictly prior to the target wake-up time. Put differently, the target wake-up time may be excluded from the time-span.

The method may further comprise: alarming the individual at an alarm time, wherein the alarm time may be at or after the target wake-up time; and wherein the act of updating the target light profile may be further based on whether the actual wake-up time is prior to, at, or after the alarm time.

The wording "alarm time" should, within the context of this disclosure, be construed as the point in time when the individual is forced to wake up, for example when an alarm goes off to wake up the individual.

Updating the target light profile further based on how the actual wake-up time relates to the alarm time may be advantageous in that it provides additional information on how the target light profile may be adjusted to improve the individual's well-being. In other words, the fact that the individual would wake up from the alarm rather than by himself/herself may suggest that the circadian rhythm need to be shifted.

The alarm time may be adjusted within a time-span around the target wake-up time, based on the circadian rhythm model of the individual, such that the alarm time is outside a deep-sleep phase of the individual. Thus, in cases where the individual is woken up by the alarm, at least he is not woken in a deep-sleep phase. Waking the individual outside of a deep-sleep phase may enhance the well-being of the individual.

The target wake-up time may be one of a plurality of wake-up times, each target wake-up time of the plurality of wake-up times being associated with a day of a plurality of consecutive days, and wherein the method may be repeated over the plurality of consecutive days.

A possible associated advantage is that the individual's circadian rhythm may be gradually shifted over the plurality of consecutive days such that the actual wake-up time and the target wake-up time may be aligned for the individual.

According to a second aspect, a control unit for adjusting an actual wake-up time of an individual is provided. The control unit comprising: circuitry configured to execute: a first determining function configured to determine a target light profile based on a target wake-up time and a circadian rhythm model of the individual, wherein the target light profile describes illuminance and spectrum of light to be received by the individual over time; a controlling function configured to control a light source configured to illuminate the individual based on the target light profile such that an accumulated amount of light received by the individual is aligned with the target light profile; a second determining function configured to determine the actual wake-up time at which the individual wakes up; and an updating function configured to, in response to the actual wake-up time being outside a predetermined time-span around the target wake-up time, update the target light profile based on a comparison of the actual wake-up time and the target wake-up time.

The updating function may be further configured to, in response to the actual wake-up time being outside a further predetermined time-span and after the target wake-up time, adjust the target wake-up time based on a comparison of the actual wake-up time and the target wake-up time, and wherein the updating of the target light profile may be based on a comparison of the actual wake-up time and the adjusted target wake-up time.

The updating function may be further configured to, in response to the actual wake-up time being outside the further predetermined time-span and prior to the target wake-up time, adjust the target wake-up time based on a comparison of the actual wake-up time and the target wake-up time, and wherein the updating of the target light profile may be based on a comparison of the actual wake-up time and the adjusted target wake-up time.

The controlling function, the second determining function and the updating function may be repeatedly executed until the actual wake-up time is within the predetermined time-span from the target wake-up time.

The predetermined time-span may be a time-span prior to the target wake-up time.

The circuitry may be further configured to execute: an alarming function configured to alarm the individual at an alarm time, wherein the alarm time may be at or after the target wake-up time, and wherein the updating function may be further configured to update the target light profile based on whether the actual wake-up time is prior to, at, or after the alarm time.

The target wake-up time may be one of a plurality of wake-up times, each target wake-up time of the plurality of wake-up times being associated with a day of a plurality of consecutive days, and wherein the functions of the circuitry may be executed repeatedly over the plurality of consecutive days.

The above-mentioned features of the first aspect, when applicable, apply to this second aspect as well. In order to avoid undue repetition, reference is made to the above.

According to a third aspect, a non-transitory computer-readable recording medium is provided. The non-transitory computer-readable recording medium having recorded thereon program code portion which, when executed at a device having processing capabilities, performs the method according to the first aspect.

The program code portions may be downloadable to the device having processing capabilities from an application providing service. The above-mentioned features of the first and second aspects, when applicable, apply to this third aspect as well. In order to avoid undue repetition, reference is made to the above.

According to a fourth aspect a system for adjusting an actual wake-up time of an individual is provided. The system comprising: a control unit according to the third aspect; and a light source communicatively connected to the control unit and configured to illuminate the individual based on the target light profile such that an accumulated amount of light received by the individual is aligned with the target light profile.

By incorporating target wake-up time information in a system (e.g. a light control system) described above, including determination of the individual's target light profile, the light control system can be operated in such a way that the circadian rhythm for the individual is adapted to the individual's needed wake up time (i.e. the individual's target wake-up time), and hence forced wake up is avoided increasing the individual's well-being.

The above-mentioned features of the first, second and third aspects, when applicable, apply to this fourth aspect as well. In order to avoid undue repetition, reference is made to the above.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

Hence, it is to be understood that this inventive concept is not limited to the particular steps of the methods described or component parts of the systems described as such method and system may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

### Brief description of the drawings

The above and other aspects of the present inventive concept will now be described in more detail, with reference to appended drawings showing variants of the present inventive concept. The figures should not be considered limiting the invention to the specific variant; instead they are used for explaining and understanding the inventive concept.

As illustrated in the figures, the sizes of layers and regions are exaggerated for illustrative purposes and, thus, are provided to illustrate the general structures of variants of the present inventive concept. Like reference numerals refer to like elements throughout.
Figure 1 schematically illustrates a system for adjusting an actual wake-up time of an individual.
Figure 2 is a schematic representation of a control unit for adjusting an actual wake-up time of an individual.
Figure 3A-C are flow charts illustrating the step of different embodiments of a method for adjusting an actual wake-up time of an individual.
Figure 4A-C shows different examples of timelines for illustrating the relationship between time-spans and points in time described in connection with Fig. 1 to 3.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

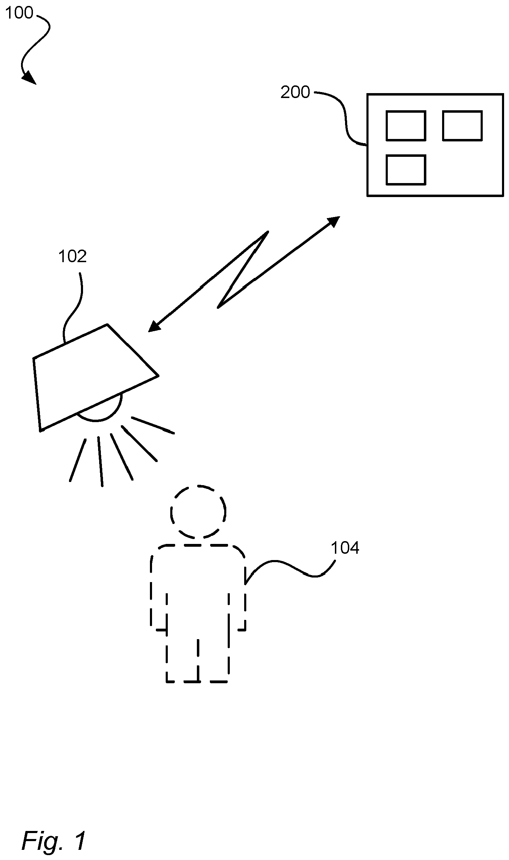

A method for adjusting an actual wake-up time of an individual, as well as a control unit and system thereof will now be described with reference to Fig. 1 to Fig. 4C.

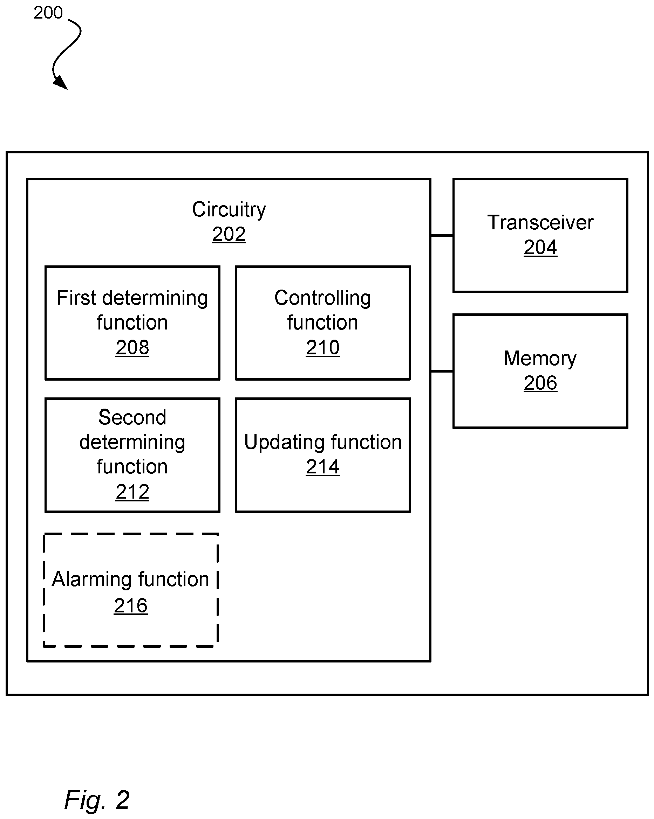

Figure 1 illustrates a system 100 for adjusting an actual wake-up time of an individual according to the present invention. The system 100 may be light control system. An example of such light control system may be a BioCentric Lighting^{™} system provided by the applicant. The system 100 comprises a control unit 200. The control unit 200 is configured to perform the control of the system 100. The control unit 200 is further described in connection with Fig. 2.

The system further comprises a light source 102 configured to illuminate the individual 104. The light source 102 is communicatively connected to the control unit 200. The light source 102 may be communicatively connected to the control unit 200 via a wired and/or a wireless connection. The wired connection may, e.g., be a connection via USB, Ethernet, Firewire, a powerline (using power line communication, PLC), etc. The wireless connection may, e.g., be a connection via Wi-Fi, Li-Fi, NFC, Bluetooth, etc. The light source 102 may comprise one or more light fixtures such as lamps. The system may further comprise a plurality of light sources configured to illuminate the individual 104.

The control unit 200 is configured to control the light source 102 to illuminate the individual 104 based on a target light profile associated with the individual 104. The light source 102 is controlled such that the illumination of the individual 104 accumulates to an amount of light that is aligned with the target light profile. In case the system comprises a plurality of light sources, the control unit 200 may be configured to control the plurality of light sources such that the illumination of the individual 104 accumulates to an amount of light that is aligned with the target light profile.

The individual 104 is herein depicted in dashed lines to illustrate that the individual 104 is not part of the system 100.

Figure 2 is a schematic illustration of the control unit 200 as described in connection with Fig. 1 above.

The control unit 200 comprises circuitry 202. The circuitry 202 may physically comprise one single circuitry device. Alternatively, the circuitry 202 may be distributed over several circuitry devices. The control unit 200 may further comprise a transceiver 204 and a memory 206. The circuitry 202 being communicatively connected to the transceiver 204 and the memory 206. The circuitry 202 may comprise a data bus (no illustrated in Fig. 2), and the circuitry 202 may communicate with the transceiver 204 and/or the memory 206 via the data bus.

The circuitry 202 may be configured to carry out overall control of functions and operations of the control unit 200. The circuitry 202 may include a processor, such as a central processing unit (CPU), microcontroller, or microprocessor. The processor may be configured to execute program code stored in the memory 206, in order to carry out functions and operations of the control unit 200.

The transceiver 204 may be configured to enable the control unit 200 to communicate with other devices. For example, the control unit 200 may collect data about the individual, such as a schedule of a calendar or wake-up times, ordinary sleep and wake cycles, and other activities affecting the circadian rhythm of the individual. This type of information may be collected from e.g. a remote server or a user device of the individual such as a phone of the individual. Further, the user may input information to the control unit, such as whether or not he woke up by himself or by an alarm. The input from the user may be used in updating of the target light profile. Including the input from the user as feedback to the method, system or control unit provides for an even more optimized target light profile.

The memory 206 may be a non-transitory computer-readable storage medium. The memory 206 may be one or more of a buffer, a flash memory, a hard drive, a removable media, a volatile memory, a non-volatile memory, a random access memory (RAM), or another suitable device. In a typical arrangement, the memory may include a non-volatile memory for long term data storage and a volatile memory that functions as system memory for the control unit 200. The memory 206 may exchange data with the circuitry 202 over the data bus. Accompanying control lines and an address bus between the memory 206 and the circuitry 202 also may be present.

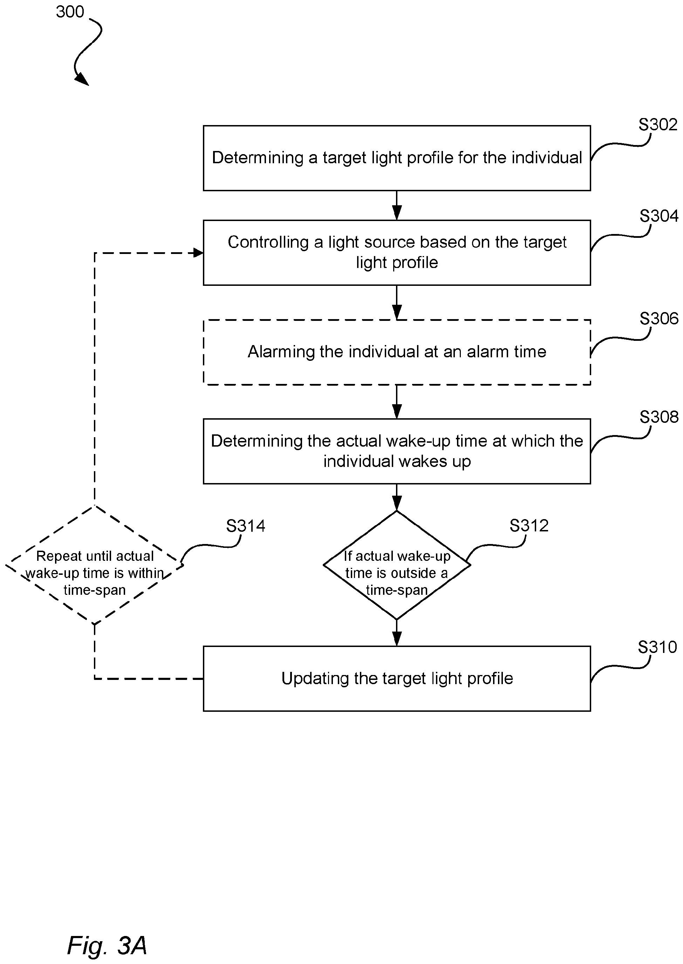

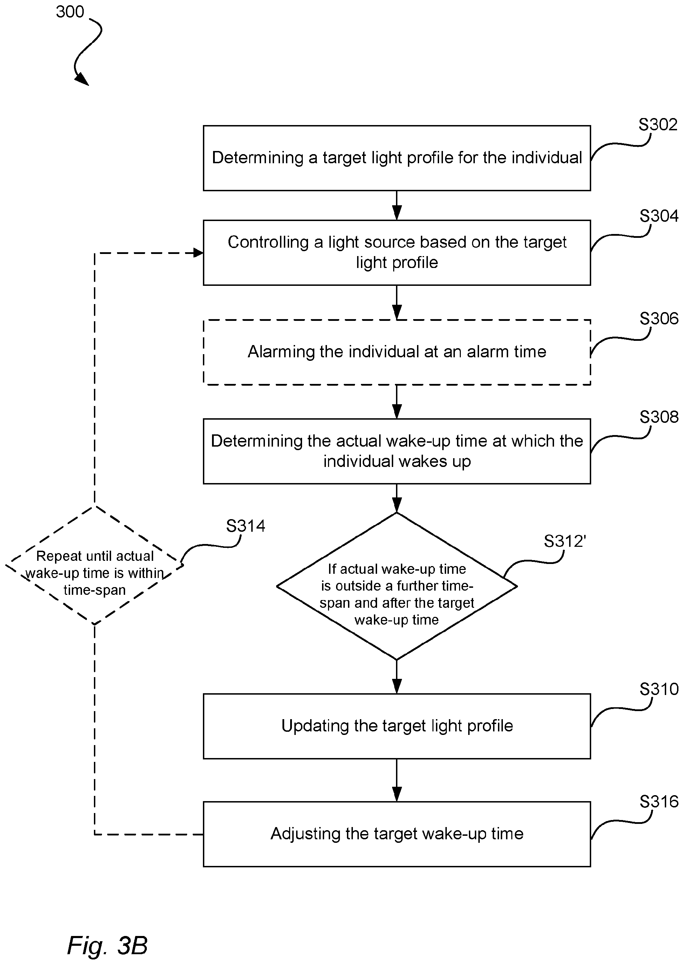

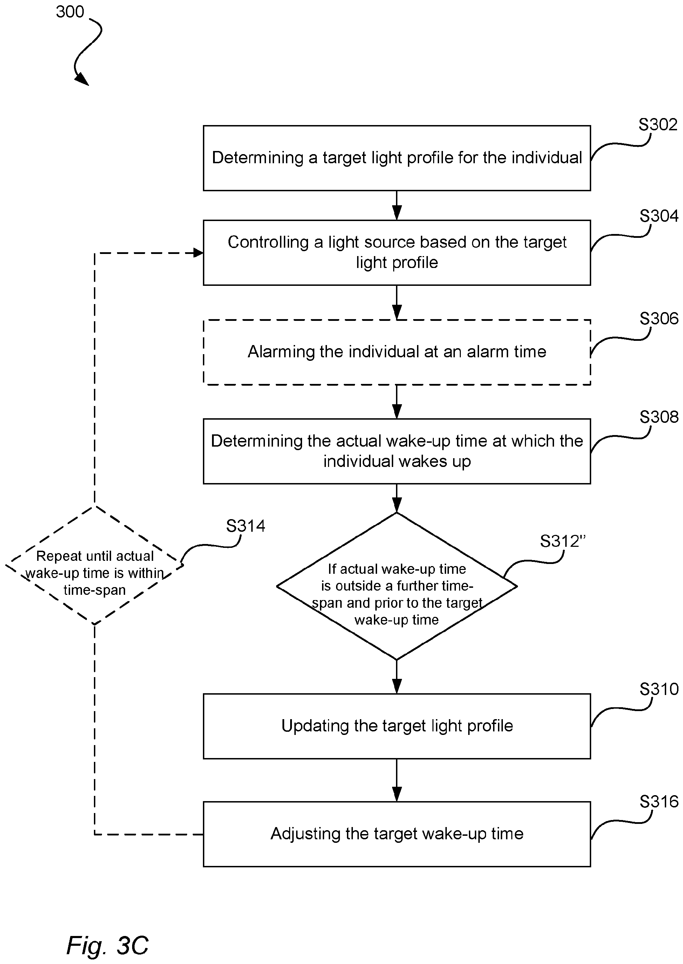

Functions and operations of the control unit 200 may be implemented in the form of executable logic routines (e.g., lines of code, software programs, etc.) that are stored on a non-transitory computer readable recording medium (e.g., the memory 206) of the control device 200 and are executed by the circuitry 202 (e.g. using the processor). Put differently, when it is stated that the circuitry 202 is configured to execute a specific function, the processor of the circuitry 202 may be configured execute program code portions stored on the memory 206, wherein the stored program code portions correspond to the specific function. Furthermore, the functions and operations of the circuitry 202 may be a stand-alone software application or form a part of a software application that carries out additional tasks related to the circuitry 202. The described functions and operations may be considered a method that the corresponding device is configured to carry out, such as the method discussed below in connection with Fig. 3. Also, while the described functions and operations may be implemented in software, such functionality may as well be carried out via dedicated hardware or firmware, or some combination of one or more of hardware, firmware, and software. The following functions may be stored on the non-transitory computer readable recording medium.

The circuitry 202 is configured to execute a first determining function 208, configured to determine a target light profile based on a target wake-up time and a circadian rhythm model of the individual, wherein the target light profile describes illuminance and spectrum of light to be received by the individual over time. More specifically, the target light profile may be determined such that it specifies a light stimulation which, when the individual is exposed to it, adjusts the circadian rhythm of the individual to match, or at least be more similar to, the target wake-up time. The target light profile may be determined based on information from the individual, such as if the person has a morning or evening chronotype. It may also be determined based on measurements (using for instance wearables) about blood pressure and/or pulse variations of the individual over the day. The measurements may further be variations in levels of hormones associated with the circadian rhythm of the individual. For example, the target light profile for an individual having an evening chronotype may be determined such that the individual, after being exposed to light according to that target light profile, should become tired earlier in the evening. Exposing that individual to light in accordance to such target light profile may result in that the individual falls asleep earlier in the evening, and may thereby wake up earlier in the morning.

The circuitry 202 is further configured to execute a controlling function 210 configured to control a light source configured to illuminate the individual based on the target light profile such that an accumulated amount of light received by the individual is aligned with the target light profile. The control unit 200 may be configured to determine the accumulated amount of light received by the individual. For instance, the control unit 200 may communicate with a light sensor configured to determine and/or estimate a light exposure of the individual. The light sensor may be worn by the individual. The light sensor may be arranged at a position which the light source is configured to illuminate. Alternatively, or additionally, the light source may be configured to communicate a current light output to the control unit 200, and the circuitry 202 may be configured to determine and/or estimate the accumulated amount of light received by the individual by accumulating the current light output over time. Alternatively, or additionally, the control unit 200 may be configured to determine and/or estimate a current light output from the light source from control instructions sent from the circuitry 202 to the light source, and the circuitry may be configured to determine and/or estimate the accumulated amount of light received by the individual by accumulating the current light output over time.

The circuitry 202 is further configured to execute a second determining function 212 configured to determine the actual wake-up time at which the individual wakes up. The actual wake-up time may be determined from data communicated to the circuitry 200 via the transceiver 204. The data may comprise information associated with the actual wake-up time of the individual. The information may, e.g., be an actual time at which the individual woke up. The data may be received by the circuitry 200 from a device configured to monitor the time at which the individual wakes up. Suitable devices may be a smartphone, a smartwatch, a smartring, an alarm clock, etc. The actual wake-up time may be information provided to the control unit 200 by the individual. For instance, the individual may enter the actual wake-up time in different device (e.g. a smartphone, etc.) which may be configured to communicate the actual wake-up time to the control unit 200, and/or directly to the control unit 200 (e.g., using a frontend). Even though not explicitly illustrated in Fig. 2, the control unit 200 may comprise input devices such as one or more of a keyboard, a mouse, and a touchscreen. The function of the first determining function 208 and the second determining function 212 may be incorporated into a single determining function.

The circuitry 202 is further configured to execute an updating function 214 configured to, in response to the actual wake-up time being outside a predetermined time-span around the target wake-up time, update the target light profile based on a comparison of the actual wake-up time and the target wake-up time. The predetermined time-span may be a time-span prior to the target wake-up time.

The updating function 214 may be further configured to, in response to the actual wake-up time being outside a further predetermined time-span and after the target wake-up time, adjust the target wake-up time based on a comparison of the actual wake-up time and the target wake-up time, and wherein the updating of the target light profile may be based on a comparison of the actual wake-up time and the adjusted target wake-up time. The updating function 214 may be further configured to, in response to the actual wake-up time being outside the further predetermined time-span and prior to the target wake-up time, adjust the target wake-up time based on a comparison of the actual wake-up time and the target wake-up time, and wherein the updating of the target light profile may be based on a comparison of the actual wake-up time and the adjusted target wake-up time.

The circuitry 202 may be further configured to execute an alarming function 216 configured to alarm the individual at an alarm time, wherein the alarm time may be at or after the target wake-up time. The updating function 214 may be further configured to update the target light profile based on whether the actual wake-up time is prior to, at, or after the alarm time.

The target wake-up time may be one of a plurality of wake-up times, each target wake-up time of the plurality of wake-up times being associated with a day of a plurality of consecutive days. The functions of the circuitry 202 may be executed repeatedly over the plurality of consecutive days.

Multiple wake-up times of the plurality of wake-up times may be used in determining or updating the target light profile. In other words, future target wake-up times may be considered when determining or updating the target light profile. For instance, the coming week's wake-up times, or just the next few day's wake-up times. This may be advantageous for instance when a future wake-up time is substantially different from other future wake-up times. For example, the individual may need to catch a flight one day and thus need to wake up earlier than usual. The target light profile may then smoothly be adjusted over a number of days in advance, in order to gradually adjust the actual wake-up time and thus the circadian rhythm of the individual.

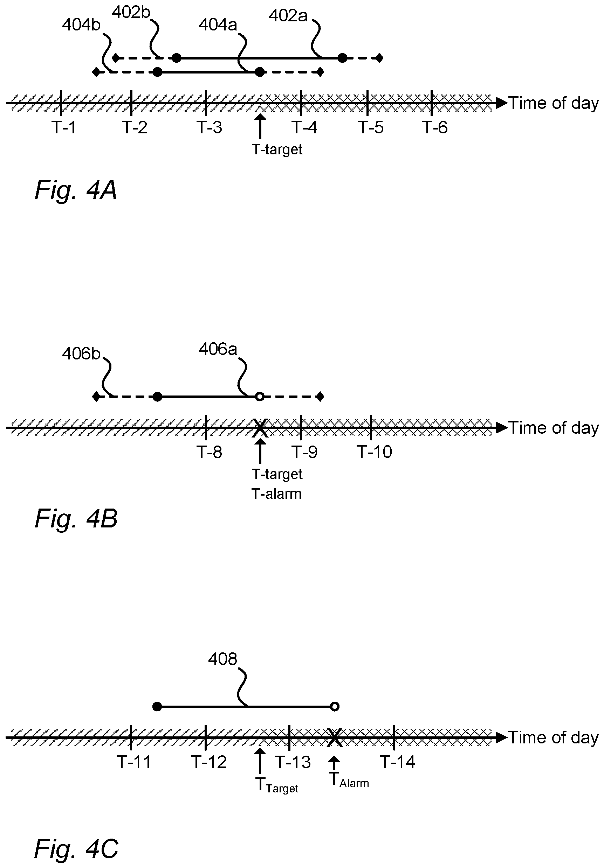

With reference to Figures 4A to 4C, the present inventive concept will now be described further with the help of illustrative examples.

In Fig. 4A, a timeline representing the time of day around a time of which the individual wakes up. The target wake-up time T-target is shown as the transition from a period when the individual is targeted to be asleep (striped pattern) and a period when the individual is targeted to be awake (crossed pattern). A first through sixth point in time, T-1 to T-6, represent different examples of actual wake-up times of the individual. Fig. 4A further illustrates two different time-spans. A first predetermined time-span 402a exemplifies how the predetermined time-span may span both before (from the left circular endpoint) and after (to the right circular endpoint) the target wake-up time. A first further predetermined time-span 402b is also shown. Herein, the first further predetermined time-span 402b is a wider than the first predetermined time-span 402a, spanning between the diamond shaped endpoints. The further predetermined time-span may span further from one end of the predetermined time span than the other as illustrated herein by the first further predetermined time-span 402b. However, it may span the same amount from both ends of the predetermined time-span. Additionally, or alternatively, the further predetermined time-span may be the same as the predetermined time-span.

Further illustrated in Fig. 4A is a second predetermined time-span 404a. As opposed to the first predetermined time-span 402a, the second predetermined time-span 404a is a time span before and up to the target wake-up time. A second further predetermined time-span 404b may extend after the target wake-up time.

Turning to the first predetermined time-span 402a and the first further predetermined time-span 402b, the updating of the target light profile may be performed according to the following. If the actual wake-up time is within the first predetermined time-span 402a, for example at the third point in time T-3 or the fourth point in time T-4, the target light profile may not be updated. The circadian rhythm in this case may be in line with the target wake-up time. If the actual wake-up time is outside the first predetermined time-span 402a, for example at the first, second, fifth or sixth point in time T-1, T-2, T-5, T-6, the target wake-up time is updated based on a comparison between the actual wake-up time and the target wake-up time T-target. The comparison may comprise determining if the individual woke up too early (e.g. at the first or second point in time T-1, T-2) or too late (e.g. at the fifth or sixth point in time T-5, T-6) and/or how much earlier or later (i.e. how far from the first pre-determined time-span 402a). The target light profile may be updated based on this information such that the actual wake-up time the following day will be closer to the target wake-up time. More specifically, if the actual wake-up time is prior to the first predetermined time-span, the target light profile may be updated so that it specifies a higher circadian stimulation of the individual later in the day. By doing so, the circadian rhythm of the individual may be shifted so that the individual gets tired later in the evening, and thus wakes up later in the morning. Further, if the actual wake-up time is after the first predetermined time-span, the target light profile may be updated so that it specifies a higher circadian stimulation of the individual earlier in the day. By doing so, the circadian rhythm of the individual may be shifted so that the individual gets tired earlier in the evening, and thus wakes up earlier in the morning.

If the individual wakes up within the first further predetermined time-span 402b (for example at the second or the fifth point in time T-2, T-5), the target light profile may be updated based on the comparison of the actual wake-up time and the target wake-up time as described above. However, if the actual wake-up time is outside the first further predetermined time-span 402b (e.g. at the first or sixth point in time T-1, T-6), the target wake-up time may need to be adjusted to aid the individual in adjusting his or her actual wake-up time such that it may become closer to the target wake-up time. This may be the case if the individual wakes up far too early or far too late compared to the target wake-up time. In the case the actual wake-up time is outside the first further predetermined time-span 402b and after the target wake-up time, e.g. the sixth point in time T-6, the target wake-up time may be adjusted based on a comparison of the actual wake-up time and the target wake-up time. For example, the adjusted target wake-up time may be shifted to a later point in time for the subsequent day. In other words, the adjusted target wake-up time may be shifted towards the point in time of the actual wake-up time The larger a difference is between the actual wake-up time and the target wake-up time, the later in time the adjusted target wake-up time may be . The adjusted target wake-up time may be limited to points in time within the first further predetermined time-span 402b. The target light profile may be updated based on a comparison of the actual wake-up time and the adjusted target wake-up time as discussed above. If the actual wake-up time is outside the first further predetermined time-span 402b and prior to the target wake-up time (e.g. at the first point in time T-1), the target wake-up time may be adjusted based on a comparison of the actual wake-up time and the target wake-up time. For example, the adjusted target wake-up time may be shifted to an earlier point in time for the subsequent day. In other words, the adjusted target wake-up time may be shifted towards the point in time of the actual wake-up time The larger the difference is between the actual wake-up time and the target wake-up time, the earlier in time the adjusted target wake-up time may be. Similar to above, the adjusted wake-up time may be limited to point in time within the first further predetermined time-span 402b. The target light profile may then be updated based on a comparison of the actual wake-up time and the adjusted target wake-up time similar to above.

In Fig. 4B, a different example of the timeline in Fig. 4A is shown. In this example, a third predetermined time-span 406a is shown, with an optional third further predetermined time-span 406b.

The later endpoint of the third predetermined time-span 406a is represented by an alarm time T-alarm. The third predetermined time-span 406a may not comprise this endpoint, which is illustrated herein by an unfilled circle. In this example, the target wake-up time T-target and the alarm time T-alarm is the same point in time.

At the alarm time T-alarm, an alarm may be sounded waking the individual. If the actual wake-up time is prior the alarm time T-alarm, e.g. an eighth point in time T-8, the target light profile may be updated (or not updated) in accordance with the discussion in connection with Fig. 4A. If the actual wake-up time is at the alarm time T-alarm (i.e. waking up from the alarm) or after the alarm time T-alarm (e.g. a ninth or tenth point in time T-9, T-10) the target light profile may be updated based on the fact that the actual wake-up time was forced, i.e. by the alarm, and/or based on a comparison between the actual wake-up time and the target wake-up time. Similar to above, if the actual wake-up time is outside a further predetermined time-span, such as the third further predetermined time-span 406b, also the target wake-up time for a subsequent day may be adjusted.

In Fig. 4C, a similar example to Fig. 4B is shown. However, in this example, the alarm time T-alarm is at a point in time after the target wake-up time T-target. A fourth predetermined time-span 408 is shown, extending on both sides of the target wake-up time T-target.

If the actual wake-up time is prior to the alarm time, and outside the fourth predetermined time-span 408 (e.g. an eleventh point in time T-11), the target light profile may be updated in a way that the individual wakes up at a later point in time the following day. However, if the actual wake-up time is prior to the alarm time and inside the predetermined time-span (e.g. a twelfth or thirteenth point in time T-12, T-13), the target light profile may not be updated. If the actual wake-up time is equal to the alarm time, i.e. the individual is woken up by the alarm, or after the alarm time (e.g. a fourteenth point in time T-14), the target light profile is updated in a way that the individual the following day wakes up at an earlier point in time.

Figure 3A is a flow chart illustrating the steps of the method 300 for adjusting an actual wake-up time of an individual.

Below, the different steps are described in more detail. Even though illustrated in a specific order, the steps of the method 300 may be performed in any suitable order, in parallel, as well as multiple times.

A target light profile for the individual is determined S302, based on a target wake-up time and a circadian rhythm model of the individual. The target light profile describes illuminance and spectrum of light to be received by the individual over time.

A light source, configured to illuminate the individual, is controlled S304 based on the target light profile such that an accumulated amount of light received by the individual is aligned with the target light profile.

The actual wake-up time at which the individual wakes up is determined S308.

In response to the actual wake-up time being outside a predetermined time-span around the target wake-up time S312, the target light profile is updated S310 based on a comparison of the actual wake-up time and the target wake-up time.

The predetermined time-span may be centered around the target wake-up time. The predetermined time-span may be a time-span prior to the target wake-up time.

Optionally, the acts of controlling S304 the light source, determining S308 the actual wake-up time and updating S310 the target light profile may be repeated S314 until the actual wake-up time is within the predetermined time-span from the target wake-up time.

The target wake-up time may be one of a plurality of wake-up times, each target wake-up time of the plurality of wake-up times being associated with a day of a plurality of consecutive days. The method may be repeated over the plurality of consecutive days.

Optionally, the individual may be alarmed S306 at an alarm time, wherein the alarm time is at or after the target wake-up time. The act of updating S310 the target light profile may be further based on whether the actual wake-up time is prior to, at, or after the alarm time.

As with the act of controlling S304 the light source, determining S308 the actual wake-up time and updating S310 the target light profile, the act of alarming S306 the individual and updating S310 the target light profile further based on whether the actual wake-up time is prior to, at, or after the alarm time, may be repeated until the actual wake-up time is within the predetermined time-span from the target wake-up time.

Fig. 3B is a flow chart illustrating the steps of an alternative of the method 300 for adjusting an actual wake-up time of an individual.

In addition to the steps mentioned above in connection with Fig. 3A, the method may further comprise the following steps. Further, any aspects mentioned in regard to the steps mention above in connection with Fig. 3A is also applicable to the embodiment illustrates herein.

In response to the actual wake-up time being outside a further predetermined time-span around the target wake-up time and after the target wake-up time S312', the method 300 may further comprise adjusting S316 the target wake-up time based on a comparison of the actual wake-up time and the target wake-up time, and wherein the updating S310 of the target light profile may be based on a comparison of the actual wake-up time and the adjusted target wake-up time.

Fig. 3C is a flow chart illustrating the steps of an alternative embodiment of the method 300 for adjusting an actual wake-up time of an individual.

In addition to the steps mentioned above in connection with Fig. 3A and 3B, the method may further comprise the following steps.

In response to the actual wake-up time being outside a further predetermined time-span around the target wake-up time and prior to the target wake-up time S312", the method 300 may further comprise adjusting S316 the target wake-up time based on a comparison of the actual wake-up time and the target wake-up time, and wherein the updating S310 of the target light profile may be based on a comparison of the actual wake-up time and the adjusted target wake-up time. The further predetermined time-span, mentioned in connection with Fig. 3B and 3C may be the same as the predetermined time-span as mentioned in connection with Fig. 3A. Alternatively, the further predetermined time span may be different from the predetermined time-span. The further predetermined time-span may be a wider time-span than the predetermined time-span.

Additionally, variations to the disclosed variants can be understood and effected by the skilled person in practicing the claimed invention, from a study of the drawings, the disclosure, and the appended claims.

## Claims

1. A method (300) for adjusting an actual wake-up time of an individual, the method (300) comprising:
determining (S302) a target light profile for the individual, based on a target wake-up time and a circadian rhythm model of the individual, wherein the target light profile describes illuminance and spectrum of light to be received by the individual over time;
controlling (S304) a light source, configured to illuminate the individual, based on the target light profile such that an accumulated amount of light received by the individual is aligned with the target light profile;
determining (S308) the actual wake-up time at which the individual wakes up; and
in response to the actual wake-up time being outside a predetermined time-span around the target wake-up time (S312):
updating (S310) the target light profile based on a comparison of the actual wake-up time and the target wake-up time.

2. The method (300) according to claim 1, further comprising:
in response to the actual wake-up time being outside a further predetermined time-span and after the target wake-up time (S312'):
adjusting (S316) the target wake-up time based on a comparison of the actual wake-up time and the target wake-up time, and wherein the updating (S310) of the target light profile is based on a comparison of the actual wake-up time and the adjusted target wake-up time.

3. The method (300) according to claim 1 or 2, further comprising:
in response to the actual wake-up time being outside the further predetermined time-span and prior to the target wake-up time (S312"):
adjusting (S316) the target wake-up time based on a comparison of the actual wake-up time and the target wake-up time, and wherein the updating (s312) of the target light profile is based on a comparison of the actual wake-up time and the adjusted target wake-up time.

4. The method (300) according to any one of claims 1-3, wherein the acts of controlling (S304) the light source, determining (S308) the actual wake-up time and updating (S310) the target light profile are repeated until the actual wake-up time is within the predetermined time-span from the target wake-up time.

5. The method (300) according to any one of claims 1-4, wherein the predetermined time-span is a time-span prior to the target wake-up time.

6. The method (300) according to any one of claims 1-5, further comprising:
alarming (S306) the individual at an alarm time, wherein the alarm time is at or after the target wake-up time; and
wherein the act of updating (S310) the target light profile is further based on whether the actual wake-up time is prior to, at, or after the alarm time.

7. The method (300) according to any one of claims 1-6, wherein the target wake-up time is one of a plurality of wake-up times, each target wake-up time of the plurality of wake-up times being associated with a day of a plurality of consecutive days, and wherein the method (300) is repeated over the plurality of consecutive days.

8. A control unit (200) for adjusting an actual wake-up time of an individual, comprising:
circuitry (302) configured to execute:
a first determining function (208) configured to determine a target light profile based on a target wake-up time and a circadian rhythm model of the individual, wherein the target light profile describes illuminance and spectrum of light to be received by the individual over time;
a controlling function (210) configured to control a light source configured to illuminate the individual based on the target light profile such that an accumulated amount of light received by the individual is aligned with the target light profile;
a second determining function (212) configured to determine the actual wake-up time at which the individual wakes up; and
an updating function (214) configured to, in response to the actual wake-up time being outside a predetermined time-span around the target wake-up time, update the target light profile based on a comparison of the actual wake-up time and the target wake-up time.

9. The control unit according to claim 8, wherein the updating function is further configured to, in response to the actual wake-up time being outside a further predetermined time-span and after the target wake-up time, adjust the target wake-up time based on a comparison of the actual wake-up time and the target wake-up time, and wherein the updating of the target light profile is based on a comparison of the actual wake-up time and the adjusted target wake-up time.

10. The control unit according to claim 8 or 9, wherein the updating function is further configured to, in response to the actual wake-up time being outside the further predetermined time-span and prior to the target wake-up time, adjust the target wake-up time based on a comparison of the actual wake-up time and the target wake-up time, and wherein the updating of the target light profile is based on a comparison of the actual wake-up time and the adjusted target wake-up time.

11. The control unit (200) according to any one of claims 8-10, wherein the controlling function (210), the second determining function (212) and the updating function (214) are repeatedly executed until the actual wake-up time is within the predetermined time-span from the target wake-up time.

12. The control unit (200) according to any one of claims 8-11, wherein the predetermined time-span is a time-span prior to the target wake-up time.

13. The control unit (200) according to any one of claims 8-12, wherein the circuitry (202) is further configured to execute:
an alarming function (216) configured to alarm the individual at an alarm time, wherein the alarm time is at or after the target wake-up time; and
wherein the updating function (214) is further configured to update the target light profile based on whether the actual wake-up time is prior to, at, or after the alarm time.

14. The control unit (200) according to any one of claims 8-13, wherein the target wake-up time is one of a plurality of wake-up times, each target wake-up time of the plurality of wake-up times being associated with a day of a plurality of consecutive days, and wherein the functions of the circuitry are executed repeatedly over the plurality of consecutive days.

15. A non-transitory computer-readable recording medium having recorded thereon program code portion which, when executed at a device having processing capabilities, performs the method (300) according to any one of claims 1-7.

16. A system (100) for adjusting an actual wake-up time of an individual (104), the system (100) comprising:
a control unit (200) according to any one of claims 8 to 14; and
a light source (102) communicatively connected to the control unit and configured to illuminate the individual based on the target light profile such that an accumulated amount of light received by the individual (104) is aligned with the target light profile.
